# EUROPEAN PATENT APPLICATION

(11) **EP 3 196 188 A1**
(43) Date of publication of application: **26.07.2017**
(21) Application number: 15842842.5
(22) Date of filing: 26.08.2015
(51) Int. Cl.: C07C 211/65, C07C 211/15, H01G 9/20

(54) **ORGANIC-INORGANIC HYBRID COMPOUND, AMINE HYDROIODIDE SALT, COMPOSITION FOR PHOTOELECTRIC CONVERSION ELEMENT, AND PHOTOELECTRIC CONVERSION ELEMENT**

(30) Priority: 16.09.2014 JP 2014188260; 16.09.2014 JP 2014188255
(71) Applicant: Kureha Corporation, Chuo-ku Tokyo 103-8552 (JP)
(72) Inventor: KANNO, Hisashi, Tokyo 103-8552 (JP)
(74) Representative: BRP Renaud & Partner mbB Rechtsanwälte Patentanwälte Steuerberater
(86) International application number: PCT/JP2015/074092
(87) International publication number: WO 2016/043002

(57) **Abstract**

The present invention provides a novel compound to be used in a solar cell. The compound of the present invention is an organic-inorganic hybrid compound represented by Formula (I).

R¹CH₂N⁺H₃M¹X¹₃ ··· (I)

where, R¹ is a C1-C5 alkyl group or C2-C5 alkenyl group substituted with at least one halogen atom; M¹ is a divalent metal ion; X¹ is a monovalent halogen atom ion; and X¹₃ is formed from one type of halogen atom ion or a combination of two or more types of halogen atom ions.

## Description

### Technical Field

The present invention relates to an organic-inorganic hybrid compound which fulfills the photoelectric conversion function of a photoelectric conversion element, a composition for forming the photoelectric conversion portion of a photoelectric conversion element, a compound for forming the composition, and a photoelectric conversion element formed using the same.

### Background Art

In recent years, there has been active research and development of solar cells (called "perovskite-type solar cells" hereafter) using compounds (called "perovskite compounds" hereafter) having an organic-inorganic hybrid-type perovskite structure (Patent Documents 1 to 3). CH₃NH₃PbX₃ (X = halogen), which is a perovskite compound, was first used in a perovskite-type solar cell in 2009 instead of a dye of a dye-sensitized solar cell (Non-Patent Document 1). Subsequently, a solid perovskite-type solar cell using only solid materials was produced in 2012, and research has been actively advanced in response to achieving a photoelectric conversion rate exceeding 10%. Subsequent research has yielded a conversion rate which surpasses that of other organic solar cells and exceeds the 17% conversion rate expected for the practical application of organic solar cells, and perovskite-type solar cells have attracted a great amount of attention as a new technology. Further, perovskite-type solar cells also have the advantages of having a simple production process and inexpensive materials in comparison to silicon-based solar cells.

It is known that the X₃ portion of a perovskite compound may be a hybrid of a plurality of halogen atoms such as I₃₋ₓClₓ (Non-Patent Document 2).

It is also known that CH₃NH₃SnX₃ (X = halogen) also has a photoelectric conversion effect (Non-Patent Document 3).

In addition, it has also been reported that the same properties are anticipated for compounds in which a methylammonium group is converted to Cs⁺ as a monovalent inorganic positive ion (Non-Patent Document 4).

It has also been reported that when existing CH₃NH₃PbX₃ is used for a photoelectric conversion element, adding an organic-inorganic hybrid compound having an ammonium group of a certain type of amino acid has a positive effect on the photoelectric conversion activity or the stability as a photoelectric conversion element (Non-Patent Document 5).

### Citation List

### Patent Literature

Patent Document 1: Japanese Patent Publication "Japanese Unexamined Patent Application Publication No. 2014-56940A" (published March 27, 2014)
Patent Document 2: Japanese Patent Publication "Japanese Unexamined Patent Application Publication No. 2014-72327A" (published April 21, 2014)
Patent Document 3: European Patent Publication No. EP2693503A1 (published February 5, 2014)

### Non-Patent Literature

Non-Patent Document 1: A. Kojima et. al., J. Am. Chem. Soc., (2009) 131, 6050-6051.
Non-Patent Document 2: S.D. Stranks et. al., Science, (2013) 342, 341-344.
Non-Patent Document 3: F. Hao et. al., Nature Photonics, (2014) 8, 489-494.
Non-Patent Document 4: C. Constantinos et. al., Inorg. Chem., (2013) 52, 9019-9038.
Non-Patent Document 5: Y. Ogomi et. al., J. Phys. Chem. C, (2014) 118, 16651-16659.

### Summary of Invention

### Technical Problem

The compound which is responsible for the photoelectric conversion of a perovskite-type solar cell is the perovskite compound CH₃NH₃PbX₃ (X = Cl, Br, or I). However, research on perovskite compounds used in perovskite-type solar cells cannot be considered to have been conducted sufficiently in the past. For example, the only known example in which a methylammonium group of CH₃NH₃PbX₃ is actually substituted with another organic group to yield relatively high activity is CH(NH₂)₂PbX₃ using formamidinium.

Therefore, the present invention was conceived in light of the problems described above, and an object thereof is to provide a new compound and material that can be used in a perovskite-type solar cell.

### Solution to Problem

In order to solve the problems described above, the organic-inorganic hybrid compound of the present invention is an organic-inorganic hybrid compound represented by Formula (I):

R¹CH₂N⁺H₃M¹X¹₃... (I)

where, R¹ is a C1-C5 alkyl group or C2-C5 alkenyl group substituted with at least one halogen atom; M¹ is a divalent metal ion; X¹ is a monovalent halogen atom ion; and X¹₃ is formed from one type of halogen atom ion or a combination of two or more types of halogen atom ions.

In order to solve the problems described above, one aspect of the composition for a photoelectric conversion element according to the present invention is a composition for a photoelectric conversion element containing the organic-inorganic hybrid compound described above.

In order to solve the problems described above, another aspect of the composition for a photoelectric conversion element according to the present invention is a composition for a photoelectric conversion element obtained by mixing an amine hydrogen halide salt represented by Formula (II):

R¹CH₂N⁺H₃X²... (II)

where, R¹ is a C1-C5 alkyl group or C2-C5 alkenyl group substituted with at least one halogen atom; and X² is a halogen atom ion
and a metal halide represented by Formula (III):

M¹X³₂... (III)

where, M¹ is a divalent metal ion; and X³ is a monovalent halogen atom ion.

In order to solve the problems described above, yet another aspect of the composition for a photoelectric conversion element according to the present invention is a composition for a photoelectric conversion element containing an amine hydrogen halide salt represented by Formula (II):

R¹CH₂N⁺H₃X²...( II)

where, R¹ is a C1-C5 alkyl group or C2-C5 alkenyl group substituted with at least one halogen atom; and X² is a halogen atom ion.

In order to solve the problems described above, the amine hydrogen iodide salt of the present invention is an amine hydrogen iodide salt represented by Formula (IIa):

R²CH₂NH₃I... (IIa)

where, R² is a C1-C5 alkyl group substituted with at least one fluorine atom.

In order to solve the problems described above, the photoelectric conversion element of the present invention is a photoelectric conversion element including a transparent electrode, a counter electrode opposing the transparent electrode, and a photoelectric conversion layer interposed between the transparent electrode and the counter electrode, the photoelectric conversion layer being any one of the layers of (a) to (c):
(a) a layer containing the organic-inorganic hybrid compound described above;
(b) a layer formed from the composition described above; and
(c) a layer formed by applying the composition described above to a metal halide represented by Formula (III):

   M¹X³₂... (III)

   where, M¹ is a divalent metal ion; and X³ is a monovalent halogen atom ion.

### Advantageous Effects of Invention

With the organic-inorganic hybrid compound and the composition for a photoelectric conversion element according to the present invention, it is possible to achieve a higher photoelectric effect than that of conventional compounds and compositions used in perovskite-type solar cells.

### Description of Embodiments

Embodiments of the organic-inorganic hybrid compound, the amine hydrogen iodide salt, the composition for a photoelectric conversion element, and the photoelectric conversion element of the present invention will be described hereinafter.

### 1. Organic-inorganic hybrid compound

The organic-inorganic hybrid compound of this embodiment is a compound represented by Formula (I) (called "organic-inorganic hybrid compound (I)" hereafter).

R¹CH₂N⁺H₃M¹X¹₃... (I)

where, R¹ is a C1-C5 alkyl group or C2-C5 alkenyl group substituted with at least one halogen atom; M¹ is a divalent metal ion; X¹ is a monovalent halogen atom ion; and X¹₃ is formed from one type of halogen atom ion or a combination of two or more types of halogen atom ions.

R¹ is a C1-C5 alkyl group or C2-C5 alkenyl group substituted with at least one halogen atom. Note that in this specification, a "Cm-Cn" hydrocarbon group refers to a hydrocarbon having from m to n carbon atoms. Examples of halogen atoms serving as substituents include fluorine atoms, chlorine atoms, bromine atoms, and iodine atoms. When the compound has two or more halogen atoms as a result of substitution, the plurality of halogen atoms may be halogen atoms of the same type as one another or halogen atoms of different types from one another. The halogen atoms serving as substituents are not particularly limited, but the compound is preferably substituted with at least one fluorine atom. In this case, when the compound has at least one fluorine atom, the C1-C5 alkyl group and C2-C5 alkenyl group may have a substitution by a halogen atom other than a fluorine atom.

On the other hand, the C1-C5 alkyl group is more preferably a C1-C4 alkyl group, even more preferably a C1-C3 alkyl group, and particularly a C1-C2 alkyl group. In addition, the C2-C5 alkenyl group is more preferably a C2-C4 alkenyl group and even more preferably a C2-C3 alkenyl group.

Accordingly, R¹ is more preferably a C1-C5 alkyl group or C2-C5 alkenyl group substituted with at least one fluorine atom, more preferably a C1-C4 alkyl group or C2-C4 alkenyl group substituted with at least one fluorine atom, even more preferably a C1-C3 alkyl group or C2-C3 alkenyl group substituted with at least one fluorine atom, particularly preferably a C1-C3 alkyl group substituted with from 1 to 5 fluorine atoms, and most preferably a C1-C2 group substituted with from 1 to 5 fluorine atoms.

Examples of C1-C5 alkyl groups substituted with at least one halogen atom include fluoromethyl groups, difluoromethyl groups, trifluoromethyl groups, chlorodifluoromethyl groups, bromodifluoromethyl groups, iododifluoromethyl groups, dichlorofluoromethyl groups, dibromofluoromethyl groups, 2-fluoroethyl groups, 2-chloro-2,2-difluoroethyl groups,
2-bromo-2,2-difluoroethyl groups, 2,2,2-trifluoroethyl groups,
1,1,2,2,2-pentafluoroethyl groups, 1,1,2,2-tetrafluoroethyl groups,
2-chloro-2,2-difluoroethyl groups, 2,2-dichloro-2-fluoroethyl groups,
2,2-dibromo-2-fluoroethyl groups, 3-fluoropropyl groups,
1,1,2,3,3,3-hexafluoropropyl groups, 1,1,2,2,3,3,3-heptafluoropropyl groups,
2,3-dibromo-2,3,3-trifluoropropyl groups, and 2,3-dichloro-2,3,3-trifluoropropyl groups. Of these, fluoromethyl groups, difluoromethyl groups, trifluoromethyl groups, 2-fluoroethyl groups, 2,2,2-trifluoroethyl groups,
1,1,2,2,2-pentafluoroethyl groups, and 1,1,2,2-tetrafluoroethyl groups are preferable; fluoromethyl groups, difluoromethyl groups, trifluoromethyl groups, 2-fluoroethyl groups, 2,2,2-trifluoroethyl groups, 1,1,2,2,2-pentafluoroethyl groups, and 1,1,2,2-tetrafluoroethyl groups are more preferable; fluoromethyl groups, difluoromethyl groups, trifluoromethyl groups, 2,2,2-trifluoroethyl groups, and 1,1,2,2,2-pentafluoroethyl groups are even more preferable; and trifluoromethyl groups, 2,2,2-trifluoroethyl groups, and
1,1,2,2,2-pentafluoroethyl groups are particularly preferable.

In addition, examples of C2-C5 alkenyl groups substituted with at least one fluorine atom include 2,2-difluoroethenyl groups, 1,2,2-trifluoroethenyl groups, 3,3-difluoro-2-propenyl groups, 2,3,3-trifluoro-2-propenyl groups, and 1,3,3,3-tetrafluoro-1-propenyl groups.

M¹ is a divalent metal ion. Examples of divalent metal ions include Pb²⁺, Sn²⁺, Sr²⁺, Cu²⁺, Zn²⁺, Mn²⁺, Fe²⁺, Ni²⁺, Co²⁺, V²⁺, Sm²⁺, Mg²⁺, Ca²⁺, Ge²⁺, Yb²⁺, Eu²⁺, Pd²⁺, and Ge²⁺. Of these, Pb²⁺, Sn²⁺, Sr²⁺, Cu²⁺, Zn²⁺, Mn²⁺, Fe²⁺, Ni²⁺, Co²⁺, V²⁺, and Sm²⁺ are preferable, and Pb²⁺ and Sn²⁺ are more preferable.

X¹ is a monovalent halogen atom ion. Examples of monovalent halogen atom ions include fluorine ions, chlorine ions, bromine ions, and iodine ions. Of these, chlorine ions, bromine ions, and iodine ions are preferable. Note that a plurality of X¹ moieties are not limited to cases in which all of the moieties are the same halogen atom ions. That is, X¹₃ is formed from one type of halogen atom ion or is formed from a combination of two or more types of halogen atom ions.

Examples of X₁³ when X₁³ is formed from a combination of two or more types of halogen atom ions include ClI₂³⁻, BrI₂³⁻, Br₂Cl³⁻, BrCl₂³⁻, Cl₂I³⁻, and Br₂I³⁻. Alternatively, there may be a combination in which all three X¹ moieties are different halogen atom ions. Further, this is not limited to cases assuming a stoichiometric coefficient, in which different types of halogen atom ions are contained at a ratio of 2:1 or 1:1:1, and the combination may be represented by X^{1a}₃₋ₛX^{1b}s (where s is a real number satisfying 0 < s < 3) or X^{1a}₃₋ₛ₋ₜX^{1b}ₛX^{1c}ₜ (where s and t are positive real numbers satisfying 0 < s + t < 3). Here, X^{1a}, X^{1b}, and X^{1c} are mutually different monovalent halogen atom ions.

A preferable aspect of the organic-inorganic hybrid compound (I) is a compound in which R¹ in Formula (I) above is a C1-C3 alkyl group substituted with from 1 to 7 fluorine atoms.

A more preferable aspect of the organic-inorganic hybrid compound (I) is a compound in which R¹ in Formula (I) above is a C1-C2 alkyl group substituted with from 1 to 5 fluorine atoms.

An even more preferable aspect of the organic-inorganic hybrid compound (I) is a compound in which R¹ in Formula (I) above is a fluoromethyl group, a difluoromethyl group, a trifluoromethyl group, a 2,2,2-trifluoroethyl group, or a 1,1,2,2,2-pentafluoroethyl group.

A particularly preferable aspect of the organic-inorganic hybrid compound (I) is a compound in which M¹ is Pb²⁺ or Sn²⁺ in each aspect described above.

The most preferable aspect of the organic-inorganic hybrid compound (I) is a compound in which, in Formula (I) above, M¹ is Pb²⁺ or Sn²⁺ and R¹ is a trifluoromethyl group, a 2,2,2-trifluoroethyl group, or a 1,1,2,2,2-pentafluoroethyl group - that is, a compound represented by Formula (Ia).

R³CH₂N⁺H₃M²X¹₃... (Ia)

In Formula (Ia), R³ is a trifluoromethyl group, a 2,2,2-trifluoroethyl group, or a 1,1,2,2,2-pentafluoroethyl group; M² is Pb²⁺ or Sn²⁺, and X¹ is the same as X¹ in Formula (I).

The organic-inorganic hybrid compound in this embodiment is presumed to have an organic-inorganic hybrid type perovskite structure, but it does not necessarily have a perovskite structure as long as it has a composition represented by Formula (I).

2. Production method for organic-inorganic hybrid compound The organic-inorganic hybrid compound (I) can be produced by reacting a hydrogen halide salt of an amine having a halogen-substituted hydrocarbon group represented by Formula (II) below (called "amine hydrogen halide salt (II)" hereafter) and a metal halide represented by Formula (III) below (called "metal halide (III)" hereafter) in a solvent.

R¹CH₂N⁺H₃X²... (II)

M¹X³₂... (III)

where, R¹ is a C1-C5 alkyl group or C2-C5 alkenyl group substituted with at least one halogen atom; and X² is a halogen atom ion. In addition, in Formula (III), M¹ is a divalent metal ion; and X³ is a monovalent halogen ion.

### Amine hydrogen halide salt (II)

The amine hydrogen halide salt (II) used in the production of the organic-inorganic hybrid compound (I) is a hydrogen halide salt of a halogen-substituted hydrocarbon amine.

R¹CH₂N⁺H₃X²... (II)

R¹ is the same as R¹ in Formula (I).

X² is a monovalent halogen atom ion. Specifically, X² is a monovalent halogen atom ion contained in X¹ in Formula (I). Therefore, examples of monovalent halogen atom ions include fluorine ions, chlorine ions, bromine ions, and iodine ions. Of these, chlorine ions, bromine ions, and iodine ions are preferable.

The amine hydrogen halide salt (II) can be produced by mixing an amine represented by Formula (IV) below (called "amine (IV)" hereafter) and HX², which is a hydrogen halide corresponding to the halogen atom ion of X², in a solvent and then distilling out the solvent.

R¹CH₂NH₂... (IV)

In Formula (IV), R¹ is the same as R¹ in formula (II).

After the solvent is distilled out, a purification step such as washing with a solvent or recrystallization may be added as necessary. The solvent used in mixing and washing is not particularly limited as long as it does not react with amines and hydrogen halides, and examples thereof include alcohols such as methanol, ethanol, and isopropanol; halogenated hydrocarbons such as dichloromethane, chloroform, and dichloroethane; aromatic hydrocarbons such as benzene, toluene, and xylene; aliphatic hydrocarbons such as petroleum ether, hexane, and methyl cyclohexane; ethers such as diethyl ether, tetrahydrofuran, and dioxane; amides such as N,N-dimethylformamide, N-methyl-2-pyrrolidinone, and N,N-dimethylacetamide; and esters such as ethyl acetate and γ-butyrolactone. Further examples include water, acetonitrile, and dimethylsulfoxide. In addition, solvents containing mixtures of two or more types thereof may also be used.

The specific conditions and the like can be set appropriately by a person skilled in the art by referencing the production method for a methyl amine hydrogen iodide salt described in Patent Document 1 or Patent Document 3 above.

In addition, when sulfates of the amines or other salts such as hydrogen halide salts of amines other than hydrogen halide salts can be acquired, the desired hydrogen halide salt can be obtained by salt exchange. Examples of methods of salt exchange that can be used include (a) a method of exchanging by neutralizing the other salt to be used for exchange with a base such as sodium hydroxide in a solvent and then reacting the resulting amine and the target hydrogen halide; and (b) a method of exchanging using an ion exchange resin.

When the amine hydrogen halide salt (II) is prepared from an amine (IV), a commercially available product may be used as the amine (IV), or a product prepared by using a halogenated compound represented by Formula (V) below (called "halogenated compound (V)" hereafter) or a sulfonic acid esterified compound of an alcohol represented by Formula (VI) below (called "sulfonic acid esterified compound (VI)" hereafter) as a raw material may be used.

R¹CH₂Z¹... (V)

R¹CH₂OZ²... (VI)

In Formula (V), R¹ is the same as R¹ in Formula (IV), and Z¹ is a halogen atom. Examples of halogen atoms include chlorine atoms, bromine atoms, and iodine atoms. In Formula (VI), R¹ is the same as R¹ in Formula (IV), and Z² is a substituted sulfonyl group. Examples of substituted sulfonyl groups include methanesulfonyl groups, phenylsulfonyl groups, p-methylphenylsulfonyl groups, trichloromethanesulfonyl groups, and trifluoromethaesulfonyl groups.

First, the halogenated compound (V) or the sulfonic acid esterified compound (VI) is reacted with a phthalimide or a carboxamide under basic conditions to obtain an N-substituted phthalimide compound or an N-substituted amide compound. Next, the phthalic acid or carboxylic acid serving as a protecting group is removed to obtain an amine (IV).

Phthalimide or the like may be used as a phthalimide. In addition, benzamide, amide acetate, or the like may be used as a carboxamide. For example, when phthalimide is used, an alkali metal salt may be prepared and then used, as in the case of phthalimide potassium.

The deprotection method is a conventionally known method, examples of which include a method of using hydrazine and a method of hydrolysis with an acid or an alkaline aqueous solution.

When Z² is a methanesulfonyl group, a p-methylphenylsulfonyl group, or the like in the sulfonic acid esterified compound (VI), the reactivity of the phthalimide and the carboxamide may be diminished due to the electron withdrawing of the R¹ group depending on the halogen atom substitution form of the R¹ group, which may cause the progression of the reaction to become slow. In this case, a substituted sulfonyl group having high electron withdrawing such as a trichloromethanesulfonyl group or a trifluoromethanesulfonyl group is preferable as Z².

The sulfonic acid esterified compound (VI) can be obtained by reacting R¹CH₂OH with a sulfonyl halide such as trichloromethanesulfonylchloride.

In addition to the method described above, an amine (IV) containing an alkyl group or an alkenyl group substituted with at least one halogen atom can be obtained by adding a hydrogen halide or a halogen molecule to an N-substituted phthalimide having an alkenyl group or an alkynyl group as a substituent and then removing the phthalic acid.

### Metal halide (III)

The metal halide (III) is a metal halide represented by Formula (III).

M¹X³₂... (III)

In Formula (III), M¹ is a divalent metal ion; and X³ is a monovalent halogen ion.

M¹ is the same as M¹ in Formula (I).

X³ is a monovalent halogen atom ion. Specifically, X³ is a monovalent halogen atom ion contained in X¹ in Formula (I). Therefore, examples of monovalent halogen atom ions include fluorine ions, chlorine ions, bromine ions, and iodine ions. Of these, chlorine ions, bromine ions, and iodine ions are preferable. X³ may be a different halogen atom ion or the same halogen atom ion as X² in the amine hydrogen halide salt (II).

### Production method

The organic-inorganic hybrid compound (I) is produced by reacting the amine hydrogen halide salt (II) and the metal halide (III) in a solvent. Here, it is presumed that a self-organization reaction due to the amine hydrogen halide salt (II) and the metal halide (III) progresses, but the reaction principle is not limited to this.

When the amine hydrogen halide salt (II) and the metal halide (III) are reacted in a solvent, the amount of the amine hydrogen halide salt (II) with respect to the metal halide (III) may be, for example, from 0.01 to 10 times the molar amount and is preferably from 0.1 to 5 times the molar amount, more preferably from 0.5 to 2 times the molar amount, and even more preferably from 0.8 to 1.2 times the molar amount.

The solvent should be one which makes it possible to uniformly mix the amine hydrogen halide salt (II) and the metal halide (III) to be reacted, and examples thereof include amides such as N,N-dimethylformamide, N,N-dimethylacetamide, and N-methyl-2-pyrrolidinone, esters such as γ-butyrolactone, and dimethylsulfoxide. Of these, when lead halide or tin halide is used as the metal halide (III), amides such as N,N-dimethylformamide, N,N-dimethylacetamide, and N-methyl-2-pyrrolidinone and solvents such as dimethylsulfoxide are particularly preferable.

The temperature when mixing the amine hydrogen halide salt (II) and the metal halide (III) is, for example, from -20 to 150°C, preferably from 0 to 100°C, and more preferably from 10 to 60°C.

The production method of the organic-inorganic hybrid compound (I) is not limited to the method described above, and the organic-inorganic hybrid compound (I) may also be produced, for example, by forming a layer of the metal halide (III) on a substrate in advance and then applying a solution containing the amine hydrogen halide salt (II) thereon so as to react the amine hydrogen halide salt (II) with the metal halide (III).

Further, the compound may also be produced by other methods such as a method using vapor deposition or a solution process using vapor.

### 3. Composition for a photoelectric conversion element

The composition for a photoelectric conversion element in this embodiment is a composition used as a material for forming a photoelectric conversion layer serving as the portion responsible for the photoelectric conversion of the photoelectric conversion element.

### First aspect of the composition for a photoelectric conversion element

A first aspect of the composition for a photoelectric conversion element is a composition for a photoelectric conversion element formed when the organic-inorganic hybrid compound (I) is dissolved or dispersed in a solvent (called "composition A for a photoelectric conversion element" hereafter).

The photoelectric conversion layer of the photoelectric conversion element can be formed by applying the composition A for a photoelectric conversion element to an object. Accordingly, the solvent for dissolving or dispersing the organic-inorganic hybrid compound (I) is not particularly limited as long as it is a solvent that can be used in coating techniques such as immersion, spin coating, and printing and as long as the solvent can dissolve the organic-inorganic hybrid compound (I) or disperse the organic-inorganic hybrid compound (I). Examples of such a solvent include halogenated hydrocarbons such as dichloromethane, chloroform, and dichloroethane; aromatic hydrocarbons such as benzene, toluene, and xylene, aliphatic hydrocarbons such as petroleum ether, hexane, and methylcyclohexane; amides such as N,N-dimethylformamide, N,N-dimethylacetamide, and N-methyl-2-pyrrolidinone; ethers such as diethyl ether, tetrahydrofuran, and dioxane; alcohols such as methanol and ethanol; and ketones such as acetone and methyl ethyl ketone. In addition to these, water, carbon disulfide, acetonitrile, ethyl acetate, γ-butyrolactone, pyridine, dimethylsulfoxide, and the like can also be used as solvents. The above solvents may be used alone, or two or more types may be mixed and used.

The content of the organic-inorganic hybrid compound (I) in the composition A for a photoelectric conversion element is, for example, from 0.01 to 90 wt.% and is preferably from 0.1 to 80 wt.%, more preferably from 1 to 60 wt.%, and even more preferably from 2 to 50 wt.%. The organic-inorganic hybrid compound (I) contained in the composition A for a photoelectric conversion element may be one type of organic-inorganic hybrid compound (I), or a plurality of types of organic-inorganic hybrid compounds (I) may be contained as a mixture.

The composition A for a photoelectric conversion element may also contain other components as long as they do not inhibit the photoelectric conversion function of the organic-inorganic hybrid compound (I). For example, in addition to the organic-inorganic hybrid compound (I), the composition A for a photoelectric conversion element may also contain CH₃N⁺H₃PbX⁴₃, CH₃N⁺H₃SnX⁴₃, CH(NH₂)(N⁺H₂)PbX⁴₃ (X⁴ is a halogen atom ion in each case), or the like, which are organic-inorganic hybrid compounds that are known to be usable in photoelectric conversion elements. In addition, compounds such as CsPbX⁴₃ and CsSnX⁴₃, in which methylammonium groups such as CH₃N⁺H₃PbX⁴₃ and CH₃N⁺H₃SnX⁴₃ are converted to monovalent inorganic positive ions, may also be contained. Here, the monovalent inorganic positive ion that is used is not particularly limited, and examples of monovalent inorganic positive ions that can be added include Li⁺, Na⁺, K⁺, Rb⁺, Cs⁺, Ag⁺, and Cu⁺. When other components other than the organic-inorganic hybrid compound (I) are contained, the ratio of the other components to the organic-inorganic hybrid compound (I) in the composition A for a photoelectric conversion element is preferably not greater than 1000 wt.%, more preferably not greater than 500 wt.%, and even more preferably not greater than 100 wt.%.

Note that a formamidinium ion refers to an ion of a form in which an H⁺ ion is added to formamidine (CH(NH₂) = NH), and several types of notation methods are possible from the resonance structure thereof, but it is described as CH(NH₂)(N⁺H₂) in this specification.

Further, an example of another component that can be contained in the composition A for a photoelectric conversion element is an organic-inorganic hybrid compound (called "organic-inorganic hybrid compound (X)" hereafter) represented by formula (X):

Q¹Y¹N⁺H₂(Y²H)M¹X¹₃... (X)

where, Q¹ is a C1-C6 alkyl group, a C3-C6 alkenyl group, or a C3-C6 alkynyl group; these groups may be substituted with halogen atoms, C1-C4 alkoxy groups, or C1-C4 alkylthio groups; M¹ is a divalent metal ion; X¹ is a monovalent halogen atom ion; a plurality of X¹ moieties may be mutually different halogen atom ions; and either Y¹ or Y² is an oxygen atom while the other is a single bond.

Q¹ is a substituted or unsubstituted C1-C6 alkyl group, C3-C6 alkenyl group, or C3-C6 alkynyl group. In addition, the substituent in the case of a substitution is a halogen atom, a C1-C4 alkoxy group, or a C1-C4 alkylthio group.

Examples of C1-C6 alkyl groups include methyl groups, ethyl groups, n-propyl groups, isopropyl groups, n-butyl groups, isobutyl groups, sec-butyl groups, n-pentyl groups, and n-hexyl groups. Of these, C1-C3 alkyl groups such as methyl groups, ethyl groups, isopropyl groups, and n-propyl groups are preferable, and methyl groups, ethyl groups, or n-propyl groups are more preferable.

Examples of C3-C6 alkenyl groups include 2-propenyl groups, 3-methyl-2-propenyl groups, 3,3-dimethyl-2-propenyl groups, and 2,4-pentadienyl. Of these, C3-C4 alkenyl groups such as 2-propenyl groups and 3-methyl-2-propenyl groups are preferable.

Examples of C3-C6 alkynyl groups include 2-propynyl groups, 3-methyl-2-propynyl groups, 3-ethyl-2-propynyl groups, and 2,4-pentadiinyl groups. Of these, C3-C4 alkynyl groups such as 2-propynyl groups and 3-methyl-2-propynyl groups are preferable.

Examples of halogen atoms serving as substituents include fluorine atoms, chlorine atoms, bromine atoms, and iodine atoms. Of these, fluorine atoms or chlorine atoms are preferable.

Examples of halogen-substituted C1-C6 alkyl groups include fluoromethyl groups, chloromethyl groups, bromomethyl groups, difluoromethyl groups, trifluoromethyl groups, 2-fluoroethyl groups, 2-bromoethyl groups, 2-iodoethyl groups, 2,2-difluoroethyl groups, 2,2,2-trifluoroethyl groups,
2-chloro-2,2-difluoroethyl groups, 2-bromo-2,2-difluoroethyl groups,
2,2-dichloro-2-fluoroethyl groups, 2,2-dibromo-2-fluoroethyl groups, 3-fluoropropyl groups, 3-chloro-3,3-difluoropropyl groups, 3-bromo-3,3-difluoropropyl groups,
3,3,3-trifluoropropyl groups, 2,2,3,3,3-pentafluoropropyl groups,
2,2,3,3-tetrafluoropropyl groups, 3-chloro-3,3-difluoropropyl groups,
3,3-dichloro-3-fluoropropyl groups, 3,3-dibromo-3-fluoropropyl groups,
4-fluorobutyl groups, 2,2,3,4,4,4-hexafluorobutyl groups,
2,2,3,3,4,4,4-heptafluorobutyl groups, 3,4-dibromo-3,4,4-trifluorobutyl groups, and
3,4-dichloro-3,4,4-trifluorobutyl groups.

Examples of halogen-substituted C3-C6 alkenyl groups include
3,3-difluoro-2-propenyl groups, 3,3-dichloro-2-propenyl groups,
3,3-dibromo-2-propenyl groups, 2,3,3-trifluoro-2-propenyl groups,
4,4-difluoro-3-butenyl groups, 3,4,4-trifluoro-3-butenyl groups, and
2,4,4,4-tetrafluoro-2-butenyl groups.

Examples of halogen-substituted C3-C6 alkynyl groups include 3-fluoro-2-propynyl groups, 3-chloro-propynyl groups, 3-bromo-2-propynyl groups, and 4,4,4-trifluoromethyl-2-butynyl groups.

Examples of C1-C4 alkoxy groups serving as substituents include methoxy groups, ethoxy groups, isopropoxy groups, n-propoxy groups, and n-butoxy groups. Of these, C1-C2 alkoxy groups such as methoxy groups or ethoxy groups are preferable.

Examples of C1-C6 alkyl groups substituted with C1-C4 alkoxy groups include methoxymethyl groups, methoxyethyl groups, methoxypropyl groups, methoxybutyl groups, methoxypentyl groups, methoxyhexyl groups, ethoxymethyl groups, ethoxyethyl groups, ethoxypropyl groups, n-propoxymethyl groups, i-propoxymethyl groups, n-propoxyethyl groups, i-propoxyethyl groups, n-propoxybutyl groups, and i-propoxybutyl groups.

Examples of C3-C6 alkenyl groups substituted with C1-C4 alkoxy groups include 3,3-dimethoxy-2-propenyl groups, 3,3-diethoxy-2-propenyl groups, and 4,4-dimethoxy-3-butenyl groups.

Examples of C3-C6 alkynyl groups substituted with C1-C4 alkoxy groups include 3-methoxy-2-propynyl groups and 3-ethoxy-2-propynyl groups.

Examples of C1-C4 alkylthio groups serving as substituents include methylthio groups, ethylthio groups, isopropylthio groups, n-propylthio groups, and n-butylthio groups. Of these, C1-C2 alkylthio groups such as methylthio groups or ethylthio groups are preferable.

Examples of C1-C6 alkyl groups substituted with C1-C4 alkylthio groups include methylthiomethyl groups, methylthioethyl groups, methylthiopropyl groups, methylthiobutyl groups, methylthiopentyl groups, methylthiohexyl groups, ethylthiomethyl groups, ethylthioethyl groups, ethylthiopropyl groups, n-propylthiomethyl groups, i-propylthiomethyl groups, n-propylthioethyl groups, i-propylthioethyl groups, n-propylthiobutyl groups, and i-propylthiobutyl groups.

Examples of C3-C6 alkenyl groups substituted with C1-C4 alkylthio groups include 3,3-di(methylthio)-2-propenyl groups,
3,3-di(ethylthio)-2-propenyl groups, and 4,4-di(methylthio)-3-butenyl groups.

Examples of C3-C6 alkynyl groups substituted with C1-C4 alkylthio groups include 3-methylthio-2-propynyl groups and 3-ethylthio-2-propynyl groups.

Of these, Q¹ is preferably a C1-C6 alkyl group or a C1-C6 alkyl group substituted with a halogen atom, a C1-C4 alkoxy group, or a C1-C4 alkylthio group, more preferably a C1-C6 alkyl group, and even more preferably a C1-C3 alkyl group.

M¹ and X¹ are respectively the same as M¹ and X¹ of the organic-inorganic hybrid compound (I).

Either Y¹ or Y² is an oxygen atom, and the other is a single bond. Accordingly, the organic-inorganic hybrid compound (X) is either a compound represented by Formula (Xa) below (called "organic-inorganic hybrid compound (Xa)" hereafter) or a compound represented by Formula (Xb) below (called "organic-inorganic hybrid compound (Xb)" hereafter).

Q¹ON⁺H₃M¹X¹₃... (Xa)

Q¹N⁺H₂(OH)M¹X¹₃... (Xb)

Here, the organic-inorganic hybrid compound (Xa) corresponds to a compound in which Y¹ is an oxygen atom and Y² is a single bond in Formula (X), and the organic-inorganic hybrid compound (Xb) corresponds to a compound in which Y¹ is a single bond and Y² is an oxygen atom in Formula (X).

Preferable aspects of the organic-inorganic hybrid compounds (Xa) and (Xb) are compounds in which, in Formulas (Xa) and (Xb) above, Q¹ is a C1-C6 alkyl group or a C1-C6 alkyl group substituted with a halogen atom, a C1-C4 alkoxy group, or a C1-C4 alkylthio group.

More preferable aspects of the organic-inorganic hybrid compounds (Xa) and (Xb) are compounds in which Q¹ is a C1-C6 alkyl group in Formulas (Xa) and (Xb) above.

More preferable aspects of the organic-inorganic hybrid compounds (Xa) and (Xb) are compounds in which Q¹ is a C1-C3 alkyl group in Formulas (Xa) and (Xb) above.

Even more preferable aspects of the organic-inorganic hybrid compounds (Xa) and (Xb) are compounds in which M¹ is Pb²⁺ or Sn²⁺ in each aspect described above.

Particularly preferable aspects of the organic-inorganic hybrid compounds (Xa) and (Xb) are compounds in which, in Formulas (Xa) and (Xb) above, M¹ is Pb²⁺ or Sn²⁺ and Q¹ is a C1-C3 alkyl group - that is, compounds represented by Formulas (Xa') and (Xb') below.

Q²ON⁺H₃M²X¹₃... (Xa')

Q²N⁺H₂(OH)M²X¹₃... (Xb')

In Formulas (Xa') and (Xb'), Q² is a C1-C3 alkyl group; M² is Pb²⁺ or Sn²⁺; and X¹ is the same as X¹ in Formula (X).

The organic-inorganic hybrid compound (X) can be produced by reacting a hydrogen halide salt of an amine having a halogen-substituted hydrocarbon group represented by Formula (XI) below (called "amine hydrogen halide salt (XI)" hereafter) and a metal halide (III) in a solvent.

Q¹Y¹N⁺H₂(Y²H)X²... (XI)

In formula (XI), Q¹, Y¹, and Y² are respectively the same as Q¹, Y¹, and Y² in formula (X). X² is the same as X² in Formula (II) described above.

The amine hydrogen halide salt (XI) is specifically a hydrogen halide salt of a hydroxyl amine having a hydrocarbon substituent on an oxygen atom represented by Formula (XIa) below, or a hydrogen halide salt of a hydroxyl amine having a hydrocarbon substituent on a nitrogen atom represented by Formula (XIb) below.

Q¹ON⁺H₃X²... (XIa)

Q¹N⁺H₂(OH)X²... (XIb)

The amine hydrogen halide salt (XI) can be produced by mixing a substituted hydroxyl amine represented by Formula (XII) below (called "amine (XII)" hereafter) and HX², which is a hydrogen halide corresponding to the halogen atom ion of X², in a solvent and then distilling out the solvent. Q¹Y¹NHY²H...(XII)

In Formula (XII), Q¹, Y¹, and Y² are respectively the same as Q¹, Y¹, and Y² in Formula (XI).

The details of the method for producing the amine hydrogen halide salt (XI) from the amine (XII) and the method for producing the organic-inorganic hybrid compound (X) from the amine hydrogen halide salt (XI) and the metal halide (III) are respectively the same as those of the method for preparing the amine hydrogen halide salt (II) and the method for producing the organic-inorganic hybrid compound (I). Accordingly, a person skilled in the art could produce the organic-inorganic hybrid compound (X) based on the above explanations of the method for producing the amine hydrogen halide salt (II) and the method for producing the organic-inorganic hybrid compound (I).

### Second aspect of the composition for a photoelectric conversion element

A second aspect of the composition for a photoelectric conversion element is a composition for a photoelectric conversion element obtained by mixing the amine hydrogen halide salt (II) and the metal halide (III) into a solvent (called "composition B for a photoelectric conversion element" hereafter).

In this aspect as well, the photoelectric conversion layer of the photoelectric conversion element can be formed by applying the composition B for a photoelectric conversion element to an object. Accordingly, the solvents indicated in the first aspect of the composition for a photoelectric conversion element can be used as the solvent used when mixing the amine hydrogen halide salt (II) and the metal halide (III). Alternatively, both components may be mixed using a solvent other than the solvents indicated in the first aspect of the composition for a photoelectric conversion element, and a solvent indicated in the first aspect of the composition for a photoelectric conversion element may be further added thereafter.

The amount of the amine hydrogen halide salt (II) with respect to the metal halide (III) may be, for example, from 0.01 to 10 times the molar amount and is preferably from 0.1 to 5 times the molar amount, more preferably from 0.5 to 2 times the molar amount, and even more preferably from 0.8 to 1.2 times the molar amount. For example, when a 2,2,2-trifluoroethylamine hydrogen iodide salt is used as the amine hydrogen halide salt (II) and lead iodide is used as the metal halide (III), as shown in Examples 2 and 13 below, although a photoelectric effect is achieved with the solar cell of Example 13 in which the amount of the amine hydrogen halide salt (II) is three times the molar amount of the metal halide (III), a better photoelectric effect can be achieved with the solar cell of Example 2 in which the amount of the amine hydrogen halide salt (II) is 1 time the molar amount of the metal halide (III).

The content of the amine hydrogen halide salt (II) in the composition B for a photoelectric conversion element is, for example, from 0.001 to 60 wt.% and is preferably from 0.1 to 50 wt.%, more preferably from 1 to 40 wt.%, and even more preferably from 2 to 30 wt.%. The content of the metal halide (III) in the composition B for a photoelectric conversion element is, for example, from 0.1 to 80 wt.% and is preferably from 0.5 to 60 wt.%, more preferably from 1 to 50 wt.%, and even more preferably from 2 to 40 wt.%. The metal halide (III) and the amine hydrogen halide salt (II) contained in the composition B for a photoelectric conversion element may respectively be one type of metal halide (III) and amine hydrogen halide salt (II), or a plurality of types of metal halides (III) or a plurality of types of amine hydrogen halide salts (II) may be mixed therein.

In this aspect as well, other components may also be contained as long as the function of the photoelectric conversion layer formed from the composition B for a photoelectric conversion element is not diminished. For example, the composition B for a photoelectric conversion element may also contain CH₃N⁺H₃PbX⁴₃, CH₃N⁺H₃SnX⁴₃, CH(NH₂)(N⁺H₂)PbX⁴₃ (X⁴ is a halogen atom ion in each case), or the like, which are organic-inorganic hybrid compounds that are known to be usable in photoelectric conversion elements. In addition, compounds such as CsPbX⁴₃ and CsSnX⁴₃, in which methylammonium groups such as CH₃N⁺H₃PbX⁴₃ and CH₃N⁺H₃SnX⁴₃ are converted to monovalent inorganic positive ions, may also be contained. Here, the monovalent inorganic positive ion that is used is not particularly limited, and examples of monovalent inorganic positive ions that can be added include Li⁺, Na⁺, K⁺, Rb⁺, Cs⁺, Ag⁺, and Cu⁺. Alternatively, CH₃N⁺H₃X⁴, CH(NH₂)(N⁺H₂)X⁴, or the like, which serve as materials for forming CH₃N⁺H₃PbX⁴₃ or CH(NH₂)(N⁺H₂)PbX⁴₃, may be further added. Similarly, halides containing monovalent inorganic positive ions such as LiX⁴, NaX⁴, KX⁴, RbX⁴, CsX⁴, AgX⁴, and CuX⁴, which serve as materials for forming compounds containing monovalent inorganic positive ions such as CsPbX⁴₃, may be added. In addition, when producing the composition B for a photoelectric conversion element by mixing the amine hydrogen halide salt (II) and the metal halide (III) into a solvent, an amine hydrogen halide salt (XI) may be further mixed therein as another component. When components other than the amine hydrogen halide salt (II) and the metal halide (III) are contained, the ratio of the other components to the total amount of the amine hydrogen halide salt (II) and the metal halide (III) added for the purpose of producing the organic-inorganic hybrid compound (I) in the composition B for a photoelectric conversion element is preferably not greater than 1,000 wt.%, more preferably not greater than 500 wt.%, and even more preferably not greater than 100 wt.%.

When the composition B for a photoelectric conversion element is used, it is presumed that photoelectric conversion is enabled by the formation of the organic-inorganic hybrid compound (I) before or after being applied to the object. However, this action mechanism is not limited as long as photoelectric conversion can be realized using the composition B for a photoelectric conversion element.

### Third aspect of the composition for a photoelectric conversion element

A third aspect of the composition for a photoelectric conversion element is a composition for a photoelectric conversion element obtained by dissolving or dispersing the amine hydrogen halide salt (II) in a solvent without containing the metal halide (III) (called "composition C for a photoelectric conversion element" hereafter). The fact that a composition containing the amine hydrogen halide salt (II) can be suitably used to form the photoelectric conversion layer of a photoelectric conversion element is new knowledge discovered by the present inventors.

In the third aspect, the photoelectric conversion layer of the photoelectric conversion element can be formed by applying the composition C for a photoelectric conversion element to a layer formed from the metal halide (III). Accordingly, the solvent for dissolving or dispersing the amine hydrogen halide salt (II) is not particularly limited as long as it is a solvent that can be used in coating techniques such as immersion, spin coating, and printing and as long as the solvent can dissolve the amine hydrogen halide salt (II) or disperse the amine hydrogen halide salt (II). The solvents indicated in the first aspect of the composition for a photoelectric conversion element can be used as such a solvent.

In addition, the composition B for a photoelectric conversion element may also be prepared by mixing the composition C for a photoelectric conversion element with the metal halide (III).

The content of the amine hydrogen halide salt (II) in the composition C for a photoelectric conversion element is, for example, from 0.01 to 90 wt.% and is preferably from 0.1 to 80 wt.%, more preferably from 0.5 to 70 wt.%, and even more preferably from 1 to 60 wt.%. The amine hydrogen halide salt (II) contained in the composition C for a photoelectric conversion element may be one type of amine hydrogen halide salt (II), or a plurality of types of amine hydrogen halide salts (II) may be mixed therein.

In the composition C for a photoelectric conversion element of this aspect as well, other components may also be contained as long as the function of the photoelectric conversion layer formed from the composition C for a photoelectric conversion element is not diminished. For example, CH₃N⁺H₃X⁴, CH(NH₂)(N⁺H₂)X⁴, or the like, which serve as materials for forming CH₃N⁺H₃PbX⁴₃ or CH(NH₂)(N⁺H₂)PbX⁴₃ (X4 is a halogen atom ion in each case) as organic-inorganic hybrid compounds known to be usable in photoelectric conversion elements, may be further added to the composition C for a photoelectric conversion element. Similarly, halides containing monovalent inorganic positive ions such as LiX⁴, NaX⁴, KX⁴, RbX⁴, CsX⁴, AgX⁴, and CuX⁴, which serve as materials for forming compounds containing monovalent inorganic positive ions such as CsPbX⁴₃, may be added. In addition, an amine hydrogen halide salt (XI) may be contained as another component. When other components other than the amine hydrogen halide salt (II) are contained, the ratio of the other components to the amine hydrogen halide salt (II) in the composition C for a photoelectric conversion element is preferably not greater than 1000 wt.%, more preferably not greater than 500 wt.%, and even more preferably not greater than 100 wt.%.

Note that in the amine hydrogen halide salt (II), the hydrogen iodide salt of a fluoro-substituted alkyl amine represented by Formula (IIa) is a novel compound that has not yet been reported.

R²CH₂NH₃I... (IIa)

(In Formula (IIa), R² is a C1-C5 alkyl group substituted with at least one fluorine atom.)

Accordingly, a hydrogen iodide salt of a fluoro-substituted alkyl amine represented by Formula (IIa) is also included in the scope of the present invention.

In Formula (IIa), examples of C1-C5 alkyls substituted with at least one fluorine atom in R² and preferable groups thereof include the C1-C5 alkyls substituted with at least one fluorine atom in R¹ and preferable groups thereof. Therefore, R² is preferably a C1-C3 alkyl group substituted with at least one fluorine atom, more preferably a C1-C2 alkyl group substituted with from 1 to 5 fluorine atoms, even more preferably a fluoromethyl group, a difluoromethyl group, a trifluoromethyl group, a 2,2,2-trifluoroethyl group, or a 1,1,2,2,2-pentafluoroethyl group, and particularly preferably a trifluoromethyl group, a 2,2,2-trifluoroethyl group, or a 1,1,2,2,2-pentafluoroethyl group.

The hydrogen iodide salt of the fluoro-substituted alkyl amine represented by Formula (IIa) can be produced with reference to the production method of the amine hydrogen halide salt (II) described above.

In addition, it has been reported that when existing CH₃NH₃PbX⁴₃ is used in a composition for a photoelectric conversion element, adding an organic-inorganic hybrid compound having an ammonium group of a certain type of amino acid has a positive effect on the photoelectric conversion activity or the stability as an element. The composition for a photoelectric conversion element of the present invention may also contain an organic-inorganic hybrid compound having an ammonium group of an amino acid or a material for forming the compound as long as the function of the photoelectric conversion layer that is formed is not diminished.

Specific examples of amino acids include glycine, alanine, 3-aminopropionic acid, 4-aminobutanoic acid, 5-aminopentanoic acid, and 6-aminohexanoic acid. The amino groups in the molecules of these amino acids are protonated to form an ammonium group.

In the mixing format of each composition, an amino acid organic-inorganic hybrid compound such as R^{x}N⁺H₃PbX⁴₃ is mixed into the composition A for a photoelectric conversion element. Here, R^{x} is a residue after an amino group is removed from the amino acid. For example, in the case of glycine, R^{x} is represented by -CH₂COOH, which bonds with the amino group at the hyphen (-) part. When an amino acid organic-inorganic hybrid compound is contained, the ratio of the amino acid organic-inorganic hybrid compound to the organic-inorganic hybrid compound (I) is preferably not greater than 100 wt.%, more preferably not greater than 50 wt.%, and even more preferably not greater than 20 wt.%.

This may be added as R^{x}N⁺H₃PbX⁴₃ to the composition B for a photoelectric conversion element, or it may be added as an amino acid hydrogen halide salt serving as a material for forming an amino acid organic-inorganic hybrid compound. When an amino acid organic-inorganic hybrid compound is contained, the ratio of the amino acid organic-inorganic hybrid compound that is added or formed inside the composition with respect to the total of the amine hydrogen halide salt (II) and the metal halide (III) in the composition B for a photoelectric conversion element is preferably not greater than 100 wt.%, more preferably not greater than 50 wt.%, and even more preferably not greater than 20 wt.%.

In addition, this may be mixed with the composition C for a photoelectric conversion element as an amino acid hydrogen halide salt serving as a material for forming an amino acid organic-inorganic hybrid compound. The ratio of the amino acid hydrogen halide salt to the amine hydrogen halide salt (II) is preferably not greater than 100 wt.%, more preferably not greater than 50 wt.%, and even more preferably not greater than 20 wt.%.

### 4. Photoelectric conversion element

The photoelectric conversion element of this embodiment includes a transparent electrode, a counter electrode opposing the transparent electrode, and a photoelectric conversion layer interposed between the transparent electrode and the counter electrode.

The photoelectric conversion element of this embodiment can function as a photoelectric conversion element and may further have a configuration that other photoelectric conversion elements may have. For example, the photoelectric conversion element of this embodiment further has a metal oxide semiconductor having a nanoporous structure and is configured so that a photoelectric conversion layer is formed on this metal oxide semiconductor. In addition, the photoelectric conversion element may have a buffer layer, a charge (electron or positive hole) transportation layer, or the like in accordance with the aspect thereof.

The photoelectric conversion element of this embodiment may be either an aspect of a wet photoelectric conversion element using an electrolyte solution containing an oxidation-reduction compound or a solid photoelectric conversion element using a charge-transportable solid material instead of an electrolyte solution.

### Transparent electrode

The material of the transparent electrode is not particularly limited, and a publicly known material may be used. Examples include electrically conductive metals such as platinum, gold, silver, copper, aluminum, indium, and titanium; electrically conductive carbons such as graphite, carbon black, carbon nanofibers, and carbon nanotubes; electrically conductive metal oxides such as fluorine-doped tin oxide (SnO₂) and zinc oxide (ZnO); and electrically conductive composite metal oxides such as indium-tin oxide (ITO) and indium-zinc oxide (IZO). Of these, a material having a high degree of transparency is preferable from the perspective of performing photoelectric conversion more efficiently. For example, fluorine-doped tin oxide (SnO₂), indium-tin oxide (ITO), or the like is more preferable.

The transparent electrode is obtained by forming a thin film of the material described above on the surface of a transparent substrate. The method for forming a thin film is not particularly limited, and sputtering, vapor deposition, a method of applying a paste dispersion, or the like may be used.

In addition, examples of transparent substrates include transparent glass substrates and transparent plastic substrates.

### Counter electrode

The materials exemplified as materials for the transparent electrode may be similarly used as materials for the counter electrode. Alternatively, sodium, sodium-potassium alloys, lithium, magnesium, aluminum, magnesium-silver mixtures, magnesium-indium mixtures, aluminum-lithium alloys, Al/Al₂O₃ mixtures, Al/LiF mixtures, or the like may also be used. Of these, electrically conductive metals such as platinum, gold, silver, copper, aluminum, indium, and titanium or electrically conductive carbons such as graphite, carbon black, carbon nanofibers, and carbon nanotubes are more preferable.

The counter electrode is obtained by directly forming a thin film of the material described above on the surface of a substrate or on the upper part of the charge transport layer or the photoelectric conversion layer. The method for forming a thin film is not particularly limited, and sputtering, vapor deposition, a method of applying a dispersion, or the like may be used.

In addition, examples of substrates include transparent glass substrates, ceramic substrates, plastic substrates, and transparent plastic substrates.

### Metal oxide semiconductor having a nanoporous structure

A metal oxide semiconductor having a nanoporous structure is a metal oxide semiconductor inside of which fine holes of a nano size are formed in a lattice shape. Metal elements used to form metal oxide semiconductors include titanium, tin, zinc, iron, tungsten, zirconium, strontium, indium, cerium, yttrium, lantham, vanadium, niobium, and tantalum. Preferable examples of metal oxide semiconductors include titanium oxide, titanium strontium oxide, zinc oxide, tin oxide, tungsten oxide, and niobium oxide. Of these, titanium oxide, zinc oxide, or tin oxide is more preferable, and titanium oxide (TiO₂) is even more preferable.

A metal oxide semiconductor having a nanoporous structure can be obtained as a solid thin film by preparing a viscous colloid or paste produced by dispersing nanoparticles of the metal oxide semiconductor described above or a precursor thereof in a solvent, applying the viscous dispersion to a transparent electrode, and then heating and baking the coating layer.

### Buffer layer

When the photoelectric conversion element is a solid photoelectric conversion element, a buffer layer is provided between the transparent electrode and the metal oxide semiconductor layer. The buffer layer is a layer which serves the role of separating the transparent electrode and the charge transport layer formed from a solid material so as to prevent electrical contact between the two components. Therefore, the buffer layer is a layer with a fine structure that does not have fine holes. Preferable materials for forming the buffer layer are metal oxides. Of metal oxides, titanium oxide, zinc oxide, niobium oxide, tin oxide, zirconium oxide, and the like are preferable, and titanium oxide is more preferable.

### Photoelectric conversion layer

A photoelectric conversion layer is formed on the surface of the metal oxide semiconductor having a nanoporous structure. The photoelectric conversion layer of this embodiment may be any one of the following (a) to (c):
(a) a layer containing the organic-inorganic hybrid compound (I);
(b) a layer formed from the composition A for a photoelectric conversion element or the composition B for a photoelectric conversion element; or
(c) a layer formed by applying the composition C for a photoelectric conversion element to the metal halide (III).

The photoelectric conversion layer can be formed from the following method (i) or (ii), for example.
(i) The composition A for a photoelectric conversion element or the composition B for a photoelectric conversion element is applied to a layer of a metal oxide semiconductor having a nanoporous structure.
(ii) A layer of a metal halide (III) is formed in advance by applying a coating agent containing the metal halide (III) to a layer of a metal oxide semiconductor having a nanoporous structure, and the composition C for a photoelectric conversion element is applied thereto.

The method used to apply the composition for a photoelectric conversion element and the coating agent containing the metal halide (III) may be a conventional coating technique such as immersion, spin coating, or printing.

### Charge transport layer

A liquid material or a solid material in accordance with the aspect of the photoelectric conversion element may be used as the charge transport layer.

A Solution which utilize the oxidation-reduction reaction between iodine ions and iodine, which are widely used in dye-sensitized solar cells, may be preferably used as liquid materials with which the charge transport layer can be formed. That is, a mixed solvent prepared by dissolving iodine and various iodide salts including inorganic salts such as sodium iodide and potassium iodide, quaternary alkylammonium salts such as tetraethylammonium iodide and benzyltrimethylammonium iodide, imidazolium salts such as 1-methyl-3-propyl-1H-imidazol-3-ium iodide, and pyridinium salts such as N-methylpyridinium iodide can be preferably used.

Examples of solid materials with which the charge transport layer can be formed include inorganic positive hole transport materials such as copper iodide and copper thiocyanate, and organic positive hole transport materials such as 2,2',7,7'-tetrakis(N,N-di(p-methoxyphenyl)amino)-9,9'-spirobifluorene(spiro-OMeT AD), poly(triarylamine), perylene, and poly(3-hexylthiophene-2,5-diyl) (P3HT). In addition, when these are used, an auxiliary agent such as lithium-bis(trifluoromethylsulfonyl)imide may be added.

### 5. Summary

As described above, the organic-inorganic hybrid compound of the present invention is an organic-inorganic hybrid compound represented by Formula (I):

R¹CH₂N⁺H₃M¹X¹₃... (I)

where, R¹ is a C1-C5 alkyl group or C2-C5 alkenyl group substituted with at least one halogen atom; M¹ is a divalent metal ion; X¹ is a monovalent halogen atom ion; and X¹₃ is formed from one type of halogen atom ion or a combination of two or more types of halogen atom ions.

In addition, in the organic-inorganic hybrid compound of the present invention, R¹ in Formula (I) above is preferably a C1-C5 alkyl group or C2-C5 alkenyl group substituted with at least one fluorine atom.

In addition, in the organic-inorganic hybrid compound of the present invention, R¹ in Formula (I) above is preferably a C1-C2 alkyl group substituted with from 1 to 5 fluorine atoms.

Further, in the organic-inorganic hybrid compound of the present invention, R¹ in Formula (I) above is preferably a fluoromethyl group, a difluoromethyl group, a trifluoromethyl group, a 2,2,2-trifluoroethyl group, or a 1,1,2,2,2-pentafluoroethyl group.

In addition, in the organic-inorganic hybrid compound of the present invention, R¹ in Formula (I) above is preferably a trifluoromethyl group, a 2,2,2-trifluoroethyl group, or a 1,1,2,2,2-pentafluoroethyl group.

Further, in the organic-inorganic hybrid compound of the present invention, M¹ in Formula (I) above is preferably Pb²⁺, Sn²⁺, Sr²⁺, Cu²⁺, Zn²⁺, Mn²⁺, Fe²⁺, Ni²⁺, Co²⁺, V²⁺, or Sm²⁺.

In addition, in the organic-inorganic hybrid compound of the present invention, M¹ in Formula (I) above is preferably Pb²⁺ or Sn²⁺.

Further, the organic-inorganic hybrid compound of the present invention is preferably an organic-inorganic hybrid compound represented by Formula (Ia):

R³CH₂N⁺H₃M²X¹₃... (Ia)

where, R³ is a trifluoromethyl group, a 2,2,2-trifluoroethyl group, or a 1,1,2,2,2-pentafluoroethyl group; M² is Pb²⁺ or Sn²⁺; and X¹ is the same as X¹ in Formula (I) above.

One aspect of the composition for a photoelectric conversion element according to the present invention is a composition for a photoelectric conversion element containing the organic-inorganic hybrid compound described above.

In addition, the aspect of the composition for a photoelectric conversion element of the present invention described above may further contain an organic-inorganic hybrid compound (called "organic-inorganic hybrid compound (X)" hereafter) represented by Formula (X):

Q¹Y¹N⁺H₂(Y²H)M¹X²₃... (X)

where, Q¹ is a C1-C6 alkyl group, a C3-C6 alkenyl group, or a C3-C6 alkynyl group; these groups may be substituted with halogen atoms, C1-C4 alkoxy groups, or C1-C4 alkylthio groups; M¹ is a divalent metal ion; X¹ is a monovalent halogen atom ion; a plurality of X¹ moieties may be mutually different halogen atom ions; and either Y¹ or Y² is an oxygen atom while the other is a single bond.

Another aspect of the composition for a photoelectric conversion element according to the present invention is a composition for a photoelectric conversion element obtained by mixing an amine hydrogen halide salt represented by Formula (II):

R¹CH₂N⁺H₃X²... (II)

where (II), R¹ is a C1-C5 alkyl group or C2-C5 alkenyl group substituted with at least one halogen atom; and X² is a halogen atom ion
and a metal halide represented by Formula (III):

M¹X³₂... (III)

where (III), M¹ is a divalent metal ion; and X³ is a monovalent halogen atom ion.

In addition, in the other aspect of the composition for a photoelectric conversion element according to the present invention described above, the amount of the amine hydrogen halide salt with respect to the metal halide is preferably from 0.5 to 2 times the molar amount.

Further, in the other aspect of the composition for a photoelectric conversion element according to the present invention described above, an amine hydrogen halide salt represented by Formula (XI):

Q¹Y¹N⁺H₂(Y²H)X²... (XI)

where, Q¹ is a C1-C6 alkyl group, a C3-C6 alkenyl group, or a C3-C6 alkynyl group; these groups may be substituted with halogen atoms, C1-C4 alkoxy groups, or C1-C4 alkylthio groups; X² is a halogen atom ion; and either Y¹ or Y² is an oxygen atom while the other is a single bond, may be further mixed therein.

Yet another aspect of the composition for a photoelectric conversion element according to the present invention is a composition for a photoelectric conversion element containing an amine hydrogen halide salt represented by Formula (II):

R¹CH₂N⁺H₃X²... (II)

where, R¹ is a C1-C5 alkyl group or C2-C5 alkenyl group substituted with at least one halogen atom; and X² is a halogen atom ion.

In addition, in yet another aspect of the composition for a photoelectric conversion element according to the present invention, R¹ in Formula (II) above is preferably a C1-C5 alkyl group or C2-C5 alkenyl group substituted with at least one fluorine atom.

Further, in yet another aspect of the composition for a photoelectric conversion element according to the present invention, R¹ in Formula (II) above is preferably a C1-C2 alkyl group substituted with from 1 to 5 fluorine atoms.

In addition, in yet another aspect of the composition for a photoelectric conversion element according to the present invention, R¹ in Formula (II) is preferably a fluoromethyl group, a difluoromethyl group, a trifluoromethyl group, a 2,2,2-trifluoroethyl group, or a 1,1,2,2,2-pentafluoroethyl group.

Further, in yet another aspect of the composition for a photoelectric conversion element according to the present invention, R¹ in Formula (II) is preferably a trifluoromethyl group, a 2,2,2-trifluoroethyl group, or a 1,1,2,2,2-pentafluoroethyl group.

In addition, yet another aspect of the composition for a photoelectric conversion element according to the present invention described above may further contain amine hydrogen halide salt represented by Formula (XI):

Q¹Y¹N⁺H₂(Y²H)X²... (XI)

where, Q¹ is a C1-C6 alkyl group, a C3-C6 alkenyl group, or a C3-C6 alkynyl group; these groups may be substituted with halogen atoms, C1-C4 alkoxy groups, or C1-C4 alkylthio groups; X² is a halogen atom ion; and either Y¹ or Y² is an oxygen atom while the other is a single bond.

The amine hydrogen iodide salt of the present invention is an amine hydrogen iodide salt represented by Formula (IIa):

R²CH₂NH₃I... (IIa)

where, R² is a C1-C5 alkyl group substituted with at least one fluorine atom.

In addition, in the amine hydrogen iodide salt of the present invention, R² in Formula (IIa) above is preferably a C1-C2 alkyl group substituted with from 1 to 5 fluorine atoms.

Further, in the amine hydrogen iodide salt of the present invention, R² in Formula (IIa) above is preferably a fluoromethyl group, a difluoromethyl group, a trifluoromethyl group, a 2,2,2-trifluoroethyl group, or a 1,1,2,2,2-pentafluoroethyl group.

In addition, in the amine hydrogen iodide salt of the present invention, R² in Formula (IIa) above is preferably a trifluoromethyl group, a 2,2,2-trifluoroethyl group, or a 1,1,2,2,2-pentafluoroethyl group.

The photoelectric conversion element of the present invention is a photoelectric conversion element including a transparent electrode, a counter electrode opposing the transparent electrode, and a photoelectric conversion layer interposed between the transparent electrode and the counter electrode, the photoelectric conversion layer being any one of the layers of (a) to (c) below:
(a) a layer containing the organic-inorganic hybrid compound described above;
(b) a layer formed from the composition described above; and
(c) a layer formed by applying the composition described above to a metal halide represented by the Formula (III):

   M¹X³₂... (III)

   where, M¹ is a divalent metal ion; and X³ is a monovalent halogen atom ion.

The production method for the composition for a photoelectric conversion element according to the present invention includes mixing an amine hydrogen halide salt represented by Formula (II):

R¹CH₂N⁺H₃X²... (II)

wherein, R¹ is a C1-C5 alkyl group or C2-C5 alkenyl group substituted with at least one halogen atom; and X² is a halogen atom ion
and a metal halide represented by Formula (III):

M¹X³₂... (III)

wherein, M¹ is a divalent metal ion; and X³ is a monovalent halogen atom ion.

The production method for a photoelectric conversion element according to the present invention is a production method for a photoelectric conversion element including a transparent electrode, a counter electrode opposing the transparent electrode, and a photoelectric conversion layer interposed between the transparent electrode and the counter electrode, the production method including forming the photoelectric conversion layer described above by any one of the following steps of (a) to (c):
(a) forming the photoelectric conversion layer using the organic-inorganic hybrid compound described above;
(b) forming the photoelectric conversion layer using the composition described above; or
(c) forming the photoelectric conversion layer by applying the composition described above to a metal halide represented by Formula (III):

   M¹X³₂ (III)

   where, M¹ is a divalent metal ion; and X³ is a monovalent halogen atom ion.

Embodiments of the present invention will be described in further detail hereinafter using examples. The present invention is not limited to the examples below, and it goes without saying that various aspects are possible with regard to the details thereof. Furthermore, the present invention is not limited to the embodiments described above, and various modifications are possible within the scope indicated in the claims. Embodiments obtained by appropriately combining the technical means disclosed by the embodiments are also included in the technical scope of the present invention. In addition, all of the documents disclosed in the present specification are hereby incorporated by reference.

### Examples

### Production of a solar cell

### Example 1

A solution for a photoelectric conversion element was prepared by measuring 2,2,2-trifluoroethylamine hydrochloride and lead iodide so that the components were equimolar and then dissolving the components in dimethylformamide (DMF) so that the total weight of the 2,2,2-trifluoroethylamine hydrochloride and the lead iodide was 20 wt.% of the entire solution.

A titanium oxide coated glass electrode (4 cm × 2 cm, titanium oxide coating part: 3 cm × 2 cm; manufactured by Nishinoda Denko) having nanoporous TiO₂ formed in advance on a glass electrode was fixed to a sample stand. While this was spun under conditions at 60 rpm for 25 seconds using a spin coater (Mikasa Spinner 1H-D2, manufactured by Mikasa Co., Ltd.), the prepared solution for a photoelectric conversion element was dropped onto the titanium oxide coated glass electrode. After dropping, the sample was spun for 60 seconds at 550 rpm or 2000 rpm. After the titanium oxide coated glass electrode coated with the solution for a photoelectric conversion element was left to stand for 10 minutes at room temperature, the electrode was dried for 2 hours at 70°C.

An electrolyte solution (ethanol solution containing sodium iodide and iodine, manufactured by Nishinoda Denko) was dropped onto the obtained electrode after drying, and a transparent electrode (4 cm × 2 cm, manufactured by Nishinoda Denko) having one surface colored black with a pencil was placed on the glass substrate onto which the electrolyte solution was dropped with the surface colored with the pencil on the inside so as to produce a solar cell (photoelectric conversion element).

### Example 2

A solar cell was produced in the same manner as in Example 1 with the exception that a 2,2,2-trifluoroethylamine hydrogen iodide salt was used instead of 2,2,2-trifluoroethylamine hydrochloride.

### Example 3

A solar cell was produced in the same manner as in Example 1 with the exception that 3,3,3-trifluoropropylamine hydrochloride was used instead of 2,2,2-trifluoroethylamine hydrochloride.

### Example 4

A solar cell was produced in the same manner as in Example 1 with the exception that 2,2,3,3,3-pentafluoropropylamine hydrochloride was used instead of 2,2,2-trifluoroethylamine hydrochloride.

### Example 5

A solar cell was produced in the same manner as in Example 1 with the exception that 2,2,2-trifluoroethylamine hydrochloride and methylamine hydrochloride were mixed and used at a molar ratio of 3:1 instead of 2,2,2-trifluoroethylamine hydrochloride.

### Example 6

A solar cell was produced in the same manner as in Example 1 with the exception that 2,2,2-trifluoroethylamine hydrochloride and formamidinium hydrochloride were mixed and used at a molar ratio of 3:1 instead of 2,2,2-trifluoroethylamine hydrochloride.

### Example 7

A solar cell was produced in the same manner as in Example 1 with the exception that 2,2,2-trifluoroethylamine hydrochloride and formamidinium hydrochloride were mixed and used at a molar ratio of 1:1 instead of 2,2,2-trifluoroethylamine hydrochloride.

### Example 8

A solar cell was produced in the same manner as in Example 1 with the exception that 2,2,2-trifluoroethylamine hydrochloride and cesium iodide were mixed at a molar ratio of 9:1 instead of 2,2,2-trifluoroethylamine hydrochloride and that DMF and dimethylsulfoxide (DMSO) were mixed and used at a weight ratio of 1:1 instead of DMF as a dissolving solvent.

### Example 9

A solar cell was produced in the same manner as in Example 1 with the exception that lead iodide and tin iodide were mixed at a molar ratio of 9:1 instead of lead iodide and that DMF and DMSO were mixed and used at a weight ratio of 1:1 instead of DMF as a dissolving solvent.

### Example 10

A solar cell was produced in the same manner as in Example 1 with the exception that a 2,2,3,3,3-pentafluoropropylamine hydrogen iodide salt was used instead of 2,2,2-trifluoroethylamine hydrochloride.

### Example 11

A solar cell was produced in the same manner as in Example 1 with the exception that 2,2,2-trifluoroethylamine hydrochloride and 5-aminovaleric acid hydrochloride (5-aminopentaoic acid hydrochloride) were mixed and used at a molar ratio of 8:2 instead of 2,2,2-trifluoroethylamine hydrochloride.

### Example 12

A solar cell was produced in the same manner as in Example 1 with the exception that a 2,2,2-trifluoroethylamine hydrogen iodide salt and a formamidinium hydrogen iodide salt were mixed and used at a molar ratio of 3:1 instead of 2,2,2-trifluoroethylamine hydrochloride.

### Example 13

A solar cell was produced in the same manner as in Example 1 with the exception that a 2,2,2-trifluoroethylamine hydrogen iodide salt was used instead of 2,2,2-trifluoroethylamine hydrochloride and that the amount of the 2,2,2-trifluoroethylamine hydrogen iodide salt that was used was 3 times the molar amount of lead iodide.

### Example 14

A solar cell was produced in the same manner as in Example 1 with the exception that 2,2,2-trifluoroethylamine hydrochloride and glycine hydrochloride were mixed and used at a molar ratio of 9:1 instead of 2,2,2-trifluoroethylamine hydrochloride.

### Example 15

A solar cell was produced in the same manner as in Example 1 with the exception that 2,2,2-trifluoroethylamine hydrochloride and methoxyamine hydrochloride were mixed and used at a molar ratio of 1:3 instead of 2,2,2-trifluoroethylamine hydrochloride.

### Example 16

A solar cell was produced in the same manner as in Example 1 with the exception that 2,2,2-trifluoroethylamine hydrochloride and methoxyamine hydrochloride were mixed and used at a molar ratio of 1:1 instead of 2,2,2-trifluoroethylamine hydrochloride.

### Example 17

A solar cell was produced in the same manner as in Example 1 with the exception that 2,2,2-trifluoroethylamine hydrochloride and methoxyamine hydrochloride were mixed and used at a molar ratio of 3:1 instead of 2,2,2-trifluoroethylamine hydrochloride.

### Comparative Example 1

A solar cell was produced in the same manner as in Example 1 with the exception that methylamine hydrochloride was used instead of 2,2,2-trifluoroethylamine hydrochloride.

### Comparative Example 2

A solar cell was produced in the same manner as in Example 1 with the exception that ethylamine hydrochloride was used instead of 2,2,2-trifluoroethylamine hydrochloride.

### Comparative Example 3

A solar cell was produced in the same manner as in Example 1 with the exception that a methylamine hydrogen iodide salt was used instead of 2,2,2-trifluoroethylamine hydrochloride.

### Comparative Example 4

A solar cell was produced in the same manner as in Example 1 with the exception that a solution for a photoelectric conversion element was not used.

### Comparative Example 5

A solar cell was produced in the same manner as in Example 1 with the exception that a formamidinium hydrogen iodide salt was used instead of 2,2,2-trifluoroethylamine hydrochloride.

### Comparative Example 6

A solar cell was produced in the same manner as in Example 1 with the exception that 5-aminovaleric acid hydrochloride was used instead of 2,2,2-trifluoroethylamine hydrochloride.

### Reference Example 1

A solar cell was produced in the same manner as in Example 1 with the exception that methoxyamine hydrochloride was used instead of 2,2,2-trifluoroethylamine hydrochloride.

### Production example of 2,2,2-trifluoroethylamine hydrogen iodide salt

The 2,2,2-trifluoroethylamine hydrogen iodide salt used in Example 2 was prepared as follows.

After 2,2,2-trifluoroethylamine (5.0 g) was dissolved in methanol (50 ml), a 57% hydriodic acid aqueous solution (7.8 ml) was added dropwise to the solution while the solution was cooled in an ice bath. After addition, the hydriodic acid remaining in the dropping funnel was washed with methanol (10 mL), and the washing liquid was also added to the solution described above. After this was stirred for approximately 10 minutes in an ice bath, the ice bath was removed, and the sample was stirred for 30 minutes. After the obtained reaction solution was condensed, an operation of adding and then removing toluene was repeated three times. After the obtained coarse solid was washed with diethyl ether, it was dried under reduced pressure to obtain the target product. Yield: 11.1 g (97%) ; melting point: 129°C

Production example of 2,2,3,3,3-pentafluoropropylamine hydrogen iodide salt The 2,2,3,3,3-pentafluoropropylamine hydrogen iodide salt used in Example 11 was prepared as follows.

After 2,2,3,3,3-pentafluoropropylamine (4.5 g) was dissolved in methanol (50 ml), a 57% hydriodic acid aqueous solution (4.0 mL) was added dropwise to the solution while the solution was cooled in an ice bath. Following addition, after this was stirred for approximately 10 minutes in an ice bath, the ice bath was removed, and the sample was stirred for 30 minutes. After the obtained reaction solution was condensed, an operation of adding and then removing toluene was repeated two times. After the obtained coarse solid was washed with toluene and diethyl ether, it was dried under reduced pressure to obtain the target product.
Yield: 7.93 g (95%) ; melting point (degradation point): approximately 260°C

### Solar cell evaluation

The produced solar cells were connected to a solar cell analyzer (manufactured by PROVA), and the short circuit current, open circuit voltage, and maximum output power were measured. Measurements were performed by irradiating each solar cell with light of the same light volume using a 300 W solar simulator (manufactured by Newport Stratford).

The results are shown in Tables 1 to 3. Table 1 shows the measurement results of cases in which a solution for a photoelectric conversion element is applied by dropping the solution for a photoelectric conversion element and then spinning the sample at 550 rpm, and Tables 2 and 3 show the measurement results of cases in which a solution for a photoelectric conversion element is applied by dropping the solution for a photoelectric conversion element and then spinning the sample at 2000 rpm. The average values of a plurality of measurements were used as measurement values.

**[Table 1]**

| | Short circuit current (mA) | Open circuit voltage (V) | Maximum output power (mW) |
|---|---|---|---|
| Example 1 | 6.2 | 0.455 | 0.550 |
| Comparative example 1 | 4.2 | 0.399 | 0.383 |
| Comparative example 2 | 2.0 | 0.384 | 0.166 |
| Comparative example 3 | 3.6 | 0.398 | 0.378 |
| Comparative example 4 | 0 | 0.251 | 0 |

**[Table 2]**

| | Short circuit current (mA) | Open circuit voltage (V) | Maximum output power (mW) |
|---|---|---|---|
| Example 1 | 6.6 | 0.416 | 0.613 |
| Example 2 | 5.3 | 0.362 | 0.415 |
| Example 3 | 5.5 | 0.432 | 0.464 |
| Example 4 | 5.9 | 0.402 | 0.531 |
| Example 5 | 6.8 | 0.409 | 0.630 |
| Example 6 | 7.1 | 0.437 | 0.666 |
| Example 7 | 6.5 | 0.449 | 0.639 |
| Example 8 | 6.4 | 0.381 | 0.547 |
| Example 9 | 6.3 | 0.439 | 0.562 |
| Example 10 | 5.6 | 0.363 | 0.447 |
| Example 11 | 6.1 | 0.470 | 0.701 |
| Example 12 | 5.8 | 0.341 | 0.431 |
| Example 13 | 1.2 | 0.300 | 0.176 |
| Example 14 | 7.0 | 0.416 | 0.594 |
| | | | |
| Comparative example 1 | 3.7 | 0.411 | 0.316 |
| Comparative example 2 | 2.0 | 0.354 | 0.225 |
| Comparative example 3 | 3.9 | 0.406 | 0.361 |
| Comparative example 5 | 1.2 | 0.401 | 0.236 |
| Comparative example 6 | 0.3 | 0.327 | 0.044 |

**[Table 3]**

| | Short-circuit current (mA) | Open circuit voltage (V) | Maximum output power (mW) |
|---|---|---|---|
| Example 1 | 6.6 | 0.416 | 0.613 |
| Example 15 | 7.7 | 0.452 | 0.748 |
| Example 16 | 7.3 | 0.438 | 0.686 |
| Example 17 | 7.4 | 0.457 | 0.690 |
| Reference Example 1 | 5.3 | 0.386 | 0.475 |

### Industrial Applicability

The present invention can be applied to technical fields related to solar cells.

## Claims

1. An organic-inorganic hybrid compound represented by Formula (I):
R¹CH₂N⁺H₃M¹X¹₃... (I)
where, R¹ is a C1-C5 alkyl group or C2-C5 alkenyl group substituted with at least one halogen atom; M¹ is a divalent metal ion; X¹ is a monovalent halogen atom ion; and X¹₃ is formed from one type of halogen atom ion or a combination of two or more types of halogen atom ions.

2. The organic-inorganic hybrid compound according to claim 1, wherein R¹ in Formula (I) is a C1-C5 alkyl group or C2-C5 alkenyl group substituted with at least one fluorine atom.

3. The organic-inorganic hybrid compound according to claim 1 or 2, wherein R¹ in Formula (I) is a C1-C2 alkyl group substituted with from 1 to 5 fluorine atoms.

4. The organic-inorganic hybrid compound according to any one of claims 1 to 3, wherein R¹ in Formula (I) is a fluoromethyl group, a difluoromethyl group, a trifluoromethyl group, a 2,2,2-trifluoroethyl group, or a 1,1,2,2,2-pentafluoroethyl group.

5. The organic-inorganic hybrid compound according to any one of claims 1 to 4,
wherein R¹ in Formula (I) is a trifluoromethyl group, a 2,2,2-trifluoroethyl group, or a 1,1,2,2,2-pentafluoroethyl group.

6. The organic-inorganic hybrid compound according to any one of claims 1 to 5,
wherein M¹ in Formula (I) is Pb²⁺, Sn²⁺, Sr²⁺, Cu²⁺, Zn²⁺, Mn²⁺, Fe²⁺, Ni²⁺, Co²⁺, V²⁺, or Sm²⁺.

7. The organic-inorganic hybrid compound according to any one of claims 1 to 6,
wherein M¹ in Formula (I) is Pb²⁺ or Sn²⁺.

8. The organic-inorganic hybrid compound according to claim 1 represented by Formula (Ia):
R³CH₂N⁺H₃M²X¹₃... (Ia)
where, R³ is a trifluoromethyl group, a 2,2,2-trifluoroethyl group, or a 1,1,2,2,2-pentafluoroethyl group; M² is Pb²⁺ or Sn²⁺; and X¹ is the same as X¹ in Formula (I).

9. A composition for a photoelectric conversion element comprising the organic-inorganic hybrid compound described in any one of claims 1 to 8.

10. The composition for a photoelectric conversion element according to claim 9 further comprising an organic-inorganic hybrid compound represented by Formula (X):
Q¹Y¹N⁺H₂(Y²H)M¹X¹₃... (X)
where, Q¹ is a C1-C6 alkyl group, a C3-C6 alkenyl group, or a C3-C6 alkynyl group; these groups may be substituted with halogen atoms, C1-C4 alkoxy groups, or C1-C4 alkylthio groups; M¹ is a divalent metal ion; X¹ is a monovalent halogen atom ion; a plurality of X¹ moieties may be mutually different halogen atom ions; and either Y¹ or Y² is an oxygen atom while the other is a single bond.

11. A composition for a photoelectric conversion element obtained by mixing an amine hydrogen halide salt represented by Formula (II):
R¹CH₂N⁺H₃X²... (II)
where, R¹ is a C1-C5 alkyl group or C2-C5 alkenyl group substituted with at least one halogen atom; and X² is a halogen atom ion
and a metal halide represented by Formula (III):
M¹X³₂... (III)
where, M¹ is a divalent metal ion; and X³ is a monovalent halogen atom ion.

12. The composition for a photoelectric conversion element according to claim 11, wherein an amount of the amine hydrogen halide salt with respect to the metal halide is from 0.5 to 2 times the molar amount.

13. The composition for a photoelectric conversion element according to claim 11 or 12, wherein an amine hydrogen halide salt represented by Formula (XI):
Q¹Y¹N⁺H₂(Y²H)X²... (XI)
where, Q¹ is a C1-C6 alkyl group, a C3-C6 alkenyl group, or a C3-C6 alkynyl group; these groups may be substituted with halogen atoms, C1-C4 alkoxy groups, or C1-C4 alkylthio groups; X² is a halogen atom ion; and either Y¹ or Y² is an oxygen atom while the other is a single bond, is further mixed therein.

14. A composition for a photoelectric conversion element comprising an amine hydrogen halide salt represented by Formula (II):
R¹CH₂N⁺H₃X²... (II)
where, R¹ is a C1-C5 alkyl group or C2-C5 alkenyl group substituted with at least one halogen atom; and X² is a halogen atom ion.

15. The composition for a photoelectric conversion element according to claim 14, wherein R¹ in Formula (II) is a C1-C5 alkyl group or C2-C5 alkenyl group substituted with at least one fluorine atom.

16. The composition for a photoelectric conversion element according to claim 14 or 15, wherein R¹ in Formula (II) is a C1-C2 alkyl group substituted with from 1 to 5 fluorine atoms.

17. The composition for a photoelectric conversion element according to any one of claims 14 to 16, wherein R¹ in Formula (II) is a fluoromethyl group, a difluoromethyl group, a trifluoromethyl group, a 2,2,2-trifluoroethyl group, or a 1,1,2,2,2-pentafluoroethyl group.

18. The composition for a photoelectric conversion element according to any one of claims 14 to 17, wherein R¹ in Formula (II) is a trifluoromethyl group, a 2,2,2-trifluoroethyl group, or a 1,1,2,2,2-pentafluoroethyl group.

19. The composition for a photoelectric conversion element according to any one of claims 14 to 18 further comprising an amine hydrogen halide salt represented by Formula (XI):
Q¹Y¹N⁺H₂(Y²H)X²... (XI)
where, Q¹ is a C1-C6 alkyl group, a C3-C6 alkenyl group, or a C3-C6 alkynyl group; these groups may be substituted with halogen atoms, C1-C4 alkoxy groups, or C1-C4 alkylthio groups; X² is a halogen atom ion; and either Y¹ or Y² is an oxygen atom while the other is a single bond.

20. An amine hydrogen iodide salt represented by Formula (IIa):
R²CH₂NH₃I... (IIa)
where, R² is a C1-C5 alkyl group substituted with at least one fluorine atom.

21. The amine hydrogen iodide salt according to claim 20, wherein R² in Formula (IIa) is a C1-C2 alkyl group substituted with from 1 to 5 fluorine atoms.

22. The amine hydrogen iodide salt according to claim 20 or 21, wherein R² in Formula (IIa) is a fluoromethyl group, a difluoromethyl group, a trifluoromethyl group, a 2,2,2-trifluoroethyl group, or a 1,1,2,2,2-pentafluoroethyl group.

23. The amine hydrogen iodide salt according to any one of claims 20 to 22, wherein R² in Formula (IIa) is a trifluoromethyl group, a 2,2,2-trifluoroethyl group, or a 1,1,2,2,2-pentafluoroethyl group.

24. A photoelectric conversion element comprising a transparent electrode, a counter electrode opposing the transparent electrode, and a photoelectric conversion layer interposed between the transparent electrode and the counter electrode, the photoelectric conversion layer being any one of the layers of (a) to (c):
(a) a layer containing the organic-inorganic hybrid compound described in any one of claims 1 to 8;
(b) a layer formed from the composition described in any one of claims 9 to 13; and
(c) a layer formed by applying the composition described in any one of claims 14 to 19 to a metal halide represented by Formula (III):
M¹X³₂... (III)
where, M¹ is a divalent metal ion; and X³ is a monovalent halogen atom ion.

25. A method for producing a composition for a photoelectric conversion element, the method comprising mixing an amine hydrogen halide salt represented by Formula (II):
R¹CH₂N⁺H₃X²... (II)
where, R¹ is a C1-C5 alkyl group or C2-C5 alkenyl group substituted with at least one halogen atom; and X² is a halogen atom ion
and a metal halide represented by Formula (III):
M¹X³₂... (III)
where, M¹ is a divalent metal ion; and X³ is a monovalent halogen atom ion.

26. A method for producing a photoelectric conversion element comprising a transparent electrode, a counter electrode opposing the transparent electrode, and a photoelectric conversion layer interposed between the transparent electrode and the counter electrode, the method comprising forming the photoelectric conversion layer by any one of the following steps of (a) to (c):
(a) forming the photoelectric conversion layer using the organic-inorganic hybrid compound described in any one of claims 1 to 8;
(b) forming the photoelectric conversion layer using the composition described in any one of claims 9 to 13; or
(c) forming the photoelectric conversion layer by applying the composition described in any one of claims 14 to 19 to a metal halide represented by Formula (III):
M¹X³₂... (III)
where, M¹ is a divalent metal ion; and X³ is a monovalent halogen atom ion.
